# EUROPEAN PATENT APPLICATION

(11) **EP 3 771 718 A1**
(43) Date of publication of application: **03.02.2021**
(21) Application number: 19305999.5
(22) Date of filing: 01.08.2019
(51) Int. Cl.: C07H 1/06, C07H 7/04, C07H 13/08, A61P 3/10

(54) **PROCESS FOR PREPARING THE CRYSTALLINE FORM II OF SOTAGLIFLOZIN**

(71) Applicant: Lexicon Pharmaceuticals, Inc., The Woodlands, TX 77381 (US)
(72) Inventor: The designation of the inventor has not yet been filed
(74) Representative: Abel & Imray

(57) **Abstract**

The present document relates to a process for the preparation of the crystalline form II of sotagliflozin, wherein said crystalline form II of sotagliflozin is directly obtained from the following compound of formula (A): 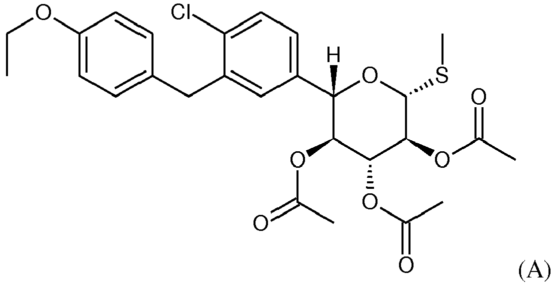 and by using toluene or xylene or mixture thereof as solvent medium for the crystallization.

## Description

The present document relates to a new process for preparing the crystalline form II of sotagliflozin.

Sotagliflozin, also called (2S,3R,4R,5S,6R)-2-(4-chloro-3-(4-ethoxybenzyl)phenyl)-6-(methylthio)tetrahydro-2H-pyran-3,4,5-triol or Methyl (5S)-5-[4-chloro-3-(4-ethoxybenzyl)phenyl]-1-thio-β-L-xylopyranoside, is a drug developed for the treatment of diabetes.

Nowadays, thirteen different forms of sotagliflozin have been identified, and in particular two anhydrous polymorph forms, named class 1 and class 4, corresponding respectively to Form I (crude sotagliflozin) and Form II (pure sotagliflozin).

These forms have been notably described in WO 2010/009197.

Form I and Form II behave like a monotropic system, the Form II being the most thermodynamically stable.

Form II is therefore the most interesting form of sotagliflozin.

With the current industrial process, Form II of sotagliflozin is produced from a methylthioether derivative in two stages, using a mixture of methanol and water as solvents during the first stage, and a mixture of methylethylketone and heptane during the second stage. The first stage corresponds to the synthesis, crystallization and isolation of Form I of sotagliflozin, and the second stage to the crystallization and isolation of Form II of sotagliflozin.

Crystalline sotagliflozin pure form II, suspended in a methylethylketone/heptane mixture, is in the form of long thin needles. As a result, the medium at the end of crystallization is difficult to stir and the cake after filtration is poorly purged and remains very moist because it still contains 50-60% of solvent. After drying, on a drying filter, sotagliflozin is in the form of very short broken needles, some forming more or less hard agglomerates.

Therefore, it remains a need of a new process for the preparation of crystalline Form II of sotagliflozin devoided from the previous drawbacks.

It also remains a need of a new process for the preparation of crystalline Form II of sotagliflozin whose industrial production cost is significantly reduced.

It also remains a need of a new process for the preparation of crystalline Form II of sotagliflozin which would be simple to implement, i.e does not request to carry out discontinuous steps.

It also remains a need of a new process for the preparation of crystalline Form II of sotagliflozin which could be advantageously performed in a continuous way with the industrial preparation of the precursor of said form II of sotagliflozin.

Thus, exemplary embodiments relate to a particular process for the preparation of the crystalline form II of sotagliflozin: 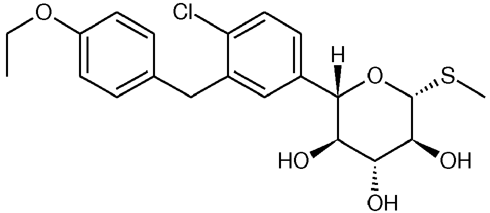 wherein said crystalline form II of sotagliflozin is directly obtained from the following compound of formula (A): 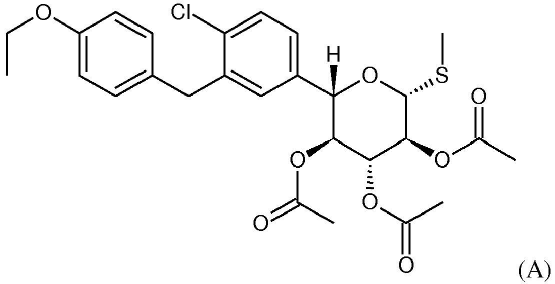 and by using toluene or xylene or mixture thereof as solvent medium for the crystallization.

Advantageously, the preparation of the crystalline form II of sotagliflozin is performed without formation of crystalline form I of sotagliflozin.

According to a preferred embodiment, the process comprises the following steps:
a) reacting the compound of formula (A) with an alcohol, in a non-aqueous solvent medium comprising a base and including at least toluene or xylene or mixture thereof, and preferably at least toluene;
b) performing an aqueous washing of said solvent medium;
c) dehydrating said solvent medium;
d) crystallizing the form II of sotagliflozin; and
e) recovering the crystalline form II of sotagliflozin.

Said process is particularly advantageous over those disclosed in the prior art since it provides Form II of sotagliflozin in good yields and with good chemical purity.

By "*good yield*", it is meant that said Form II of sotagliflozin is obtained in a yield higher than or equal to 90%.

As used herein a "*good chemical purity*" is a purity which is higher than or equal to 99%.

Indeed, the process advantageously allows to achieve a clean synthesis. There are no side reactions or degradation in the synthesis leading to the formation of impurities.

Furthermore, such process can be advantageously continuously performed.

Thus, the crystalline form II of sotagliflozin may be synthetized in a batch process or in a continuous process.

In particular, the crystalline form II of sotagliflozin is synthetized in a batch process.

Preferably, the crystalline form II of sotagliflozin is synthetized in a continuous process.

"Continuous process" means a process which can be continued without interruption for feeding and/or removal of involved products, as distinct from "batch process".

### Synthesis of sotagliflozin

Sotagliflozin is synthetized from the following compound of formula (A):

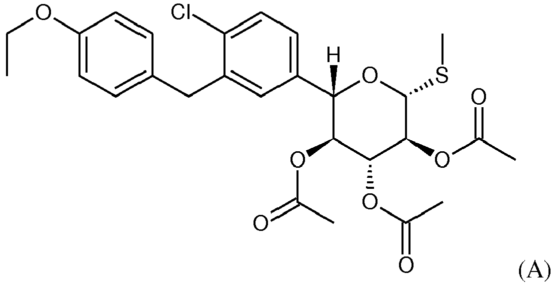

This compound (A) is dissolved in a non-aqueous solvent medium including at least toluene or xylene or mixture thereof, and preferably at least toluene.

Toluene or xylene are particularly advantageous since they allow the solubilization of the compound (A) and of sotagliflozin.

In a preferred embodiment, the compound (A) reacts with an alcohol to form sotagliflozin.

Said alcohol may be chosen for example from methanol, ethanol, 1-propanol, 2-propanol, butanol, pentanol or hexadecanol, in particular from lower alcohol such as methanol, ethanol, 1-propanol or 2-propanol, preferably from methanol and ethanol, and more preferably is methanol.

The content of alcohol is at least equal to a catalytic quantity, preferably is higher than 12 equivalents, and more preferably is from 12 to 20 equivalents.

In a preferred embodiment, the synthesis of crystalline form II of sotagliflozin is performed at a temperature below the boiling point of methanol. The boiling point of methanol at atmospheric pressure (760 mm Hg) is 64.7°C.

In particular, the synthesis is performed at a temperature below 65°C, and preferably below 63°C, at the atmospheric pressure.

More particularly, it is performed at a temperature from 60°C to 65°C, and preferably from 60°C to 63°C, at the atmospheric pressure. More preferably, the synthesis is performed at 60°C.

Such temperature may be higher in case the synthesis is performed under high pressure. For example, the temperature may be around 110°C if the pressure is at 7-8 bar.

In a preferred embodiment, a first solution of compound (A) in toluene or in xylene or in a mixture of toluene and xylene is prepared. This solution may be heated to completely dissolve compound (A), in particular at a temperature from 25°C to 45°C, and preferably from 30°C to 40°C.

Preferably, according to this embodiment, a first solution of compound (A) in toluene is prepared.

The content of toluene may be from 5 to 19 Volumes, in particular from 10 to 19 Volumes, preferably from 10 to 15 Volumes, and more preferably may be of 10 Volumes.

To perform the synthesis of sotagliflozin from compound (A), the non-aqueous solvent medium also advantageously includes a base, in particular chosen from sodium hydroxide, sodium methoxide or sodium ethoxide, and preferably the base is sodium methoxide.

The content of base(s) is at least equal to a catalytic quantity, in particular higher than 0.5 equivalent, and preferably higher than 0.6 equivalent.

Preferably, the non-aqueous solvent medium comprises sodium methoxide (MeONa) and methanol (MeOH).

According to this variant, and when the non-aqueous solvent medium comprises toluene, the concentration of compound (A) may be comprised from 10 Volumes to 19 Volumes of toluene, and preferably may be equal to 10 Volumes of toluene.

When the synthesis of sotagliflozin from compound (A) is performed with a non-aqueous solvent medium comprising toluene, the solution of compound (A) in toluene is advantageously complemented with a solution of MeONa and MeOH.

In this purpose, a solution of MeONa and MeOH may be prepared which content of MeONa is at least equal to a catalytic quantity, in particular higher than 0.5 equivalent, preferably higher than 0.6 equivalent, and the content of MeOH is at least equal to a catalytic quantity, and preferably is higher than 12 equivalents.

In particular, the content of MeONa is from a catalytic quantity to 1 equivalent, in particular from 0.5 to 1 equivalent, preferably from 0.6 to 1 equivalent, and the content of MeOH is from 12 to 20 equivalents. For example, it can be used 0.6 equivalent of MeONa and 15 equivalents of MeOH.

"Catalytic quantity" means that the material, such as MeONa or MeOH, is used in a small amount relative to the starting material, i.e. compound (A), but enough to perform the reaction.

Said solution of MeONa / MeOH may also be preheated before to be admixed to the solution of compound (A) in toluene.

To perform the synthesis, a continuous stirred-tank reactor (CSTR) may be used.

Preferably, the synthesis is performed under stirring.

The stirring speed may be set by a skilled man through different routine experiments. It notably depends on the volume of the chamber used.

For example, for a reactor with a volume below 1 liter, the stirring speed may be 500 tr/min.

### Aqueous washing

An aqueous washing may be contemplated after the synthesis of sotagliflozin from the compound A.

Preferably, the aqueous washing of the medium is performed with 0.5 Volume to 1 Volume of water.

The temperature during aqueous washing may be from 55°C to 65°C, and for example be 60°C. Such temperature is advantageous to avoid an intermediate cooling and to prevent the crystallization at this stage of the process.

This step may be performed with a mixer/settler.

Preferably, the aqueous washing is performed under stirring.

For example, the stirring may be 450 tr/min.

### Dehydration

When an aqueous washing is performed, a following step of dehydration is considered.

Such a dehydration step is advantageous for preventing the formation of hydrates of sotagliflozin, for avoiding loss of sotagliflozin for example in methyl acetate and/or methanol in which it is soluble and to remove if present residual solvents, such as methyl acetate and methanol.

The dehydration is preferably performed by evaporation.

The dehydration may be performed under atmospheric pressure or under reduced pressure.

For example, a falling film evaporator may be used.

Preferably, at least 10% v/v, and preferably at least 15% v/v of the solution is evaporated.

Advantageously, the content of water in the medium after dehydration is less than 300 ppm.

Further, after evaporation, the medium is advantageously substantially free of methyl acetate and of MeOH.

Preferably, before crystallization, the concentration of the toluenic solution may be adjusted from 40 g/l to 80 g/l, preferably from 45 g/l to 50 g/l, and more preferably to 50 g/l.

### Crystallization

Thanks to the described process, crystalline Form II of sotagliflozin is directly crystallized without formation of Form I of sotagliflozin.

Furthermore, the losses of synthetized crystalline Form II of sotagliflozin in the mother liquors are very low.

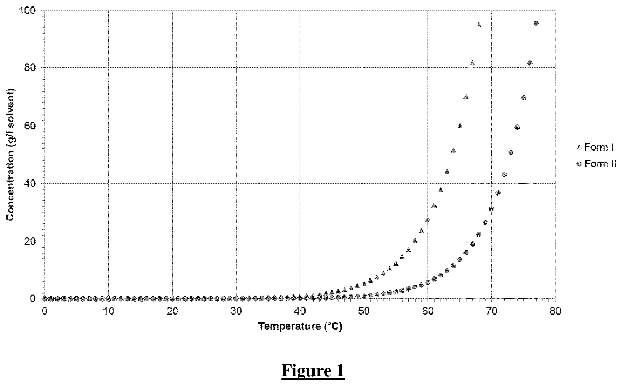

The crystallization of sotagliflozin is in particular performed at the temperature of the crystallization of form II of sotagliflozin. The temperature of the crystallization depends on the concentration. It may be chosen based on solubility curves of forms I and II of sotagliflozin as illustrated in Figure 1.

Preferably, it is performed at a temperature from 60°C to 70°C, preferably from 62°C to 67°C, and more preferably at 65°C, at the atmospheric pressure.

In particular, when the non-aqueous solvent medium comprises toluene, the content of toluene used during the crystallization may be from 40 g/l to 80 g/l.

According to a preferred embodiment, the formation of the crystalline form II of sotagliflozin is initiated with pre-existing crystalline form II of sotagliflozin. Such pre-existing crystalline form II of sotagliflozin may be synthetized, for example, following the process described in WO 2010/009197.

In particular, the formation of the crystalline form II of sotagliflozin may be initiated with 2% to 15% w/w of pre-existing form II of sotagliflozin, and preferably with 2% to 10% w/w of pre-existing form II of sotagliflozin.

During crystallization, it may be advantageous to implement a wet milling of the medium to stir it easily.

To perform the crystallization, a continuous stirred-tank reactor (CSTR) may be advantageously used.

According to a preferred embodiment, the crystallization is performed in two steps. In the first step, the solution of sotagliflozin is heated to a temperature from 60°C to 70°C, preferably from 62°C to 67°C, and more preferably at 65°C, at the atmospheric pressure. In the second step, the obtained suspension is cooled at a temperature from 20°C to 30°C, preferably from 20°C to 25°C, and more preferably at a temperature of 20°C.

In this variant, a wet milling may be performed during the first step.

According to this variant, the crystallisation of sotagliflozin may be performed in a cascade of two or more continuous stirred-tank reactors.

Preferably, the crystallisation of sotagliflozin is performed in a cascade of two continuous stirred-tank reactors.

### Filtration and washing

The crystalline form II of sotagliflozin may be recovered by filtration and washing, preferably with toluene or xylene or mixture thereof, and more preferably with toluene.

During this step, mother and wash liquors are removed.

In particular, the filtration of the mother liquors may be performed at a temperature below 40°C, in particular below 30°C, and preferably at 20°C.

Preferably, the resulting wet cake is washed with 2 Volumes of toluene at 25°C.

The filtration may be carried out under vacuum or under high pressure. Preferably, the filtration is carried out under high pressure, for example at 3 bar.

For example, the filtration may be performed on sintered or on filtration cell.

In a continuous process, the filtration may be performed on a continuous filter, for example on a rotary pressure filter or on a vacuum band filter.

The washing is carried out with a solvent, for example with toluene or xylene or mixture thereof, preferably with toluene.

The filtration and washing may be followed with a drying step.

### Drying

In particular, a drying step is performed at a temperature from 45°C to 65°C, and in particular from 50°C to 55°C.

The drying step may be carried out under a pressure below 100 mbar, and in particular below 50 mbar.

The drying step may be performed in a vacuum oven.

A conical screw dryer or a pallet dryer may also be used.

### Calibration

In particular, the calibration is carried out at a temperature from 20°C to 30°C, and preferably at 25°C.

In particular, a conical sieve mill may be used.

This step allows the elimination of clusters formed during the drying step.

The examples that follow describe the preparation of Form II of sotagliflozin. These examples are not limiting and serve merely to illustrate the process.

### EXAMPLES

### Example 1: Batch process of preparation of crystalline form II of sotagliflozin with toluene

### Synthesis of sotagliflozin in toluene

Sotagliflozin was synthetized in a batch reactor at a temperature of 60°C under atmospheric pressure with 10 Volumes of toluene, 0.6 equivalent of MeONa and 12 equivalents of MeOH. The content in compound (A) was equal to 30 g in 10 Volumes of toluene.

The time of residence in the batch reactor was 15 minutes.

Sotagliflozin was synthetized with a yield of 99.5%.

### Aqueous washing

An aqueous wash of the reaction medium was performed at 60°C with 1 Volume of water. Sodium salts were well removed.

### Dehydration

The medium was dehydrated by evaporation of 15% v/v of the solution.

### Crystallization

The crystallisation of sotagliflozin was performed at 67°C by seeding with 7.5% w/w of form II of sotagliflozin and cooling until 40°C.

### Filtration

A filtration of the suspension was performed at 40°C on filtration cell.

### Washing

A wash of the medium was performed at 25°C with 2 Volumes of toluene.

### Drying

A drying of the medium was then performed at 50°C in a vacuum oven.

Form II of sotagliflozin was crystallized with a yield of 92%.

An XRPD (X-ray diffraction) analysis confirmed that Form II of sotagliflozin was obtained.

### Example 2: Continuous process of preparation of crystalline form II of sotagliflozin with toluene

### Synthesis of sotagliflozin in toluene

Sotagliflozin was synthetized in a continuous stirred-tank reactor with a volume reactor of 230 mL, and a stirring at 500 tr/min.

450 g of compound (A) was used with 10 Volumes of Toluene to prepare a solution of compound (A) in toluene of 92.3 g/l (893 kg/m³). The reactor was fed with this solution with a flow of 372 g/h.

0.6 equivalent of MeONa and 15 equivalents of MeOH was used to prepare a methanolic solution of MeONa of 828 kg/m³. The reactor was fed with this solution with a flow of 35.8 g/h.

The synthesis was performed at 60 - 61°C.

The time of residence in the reactor was 30 minutes.

Sotagliflozin was synthetized with a yield of 98%.

### Aqueous washing

An aqueous wash of the reaction medium was performed with a mixer (264 ml) and a settler (631 ml), under stirring at 450 tr/min.

The wash was performed with 1 Volume of water / reaction medium, with a flow of 460 g/h, at 61°C.

The time of residence in the mixer was 15 minutes.

The time of residence in the settler was 45 minutes.

Sodium salts were well removed.

### Dehydration

The medium was dehydrated by evaporation of 15% v/v of the solution at 67-109°C. After evaporation, the content of MeOH was 0% w/w, the content of AcOMe was 0.15% w/w and the content of water was 140 ppm.

The concentration of the toluenic solution of sotagliflozin was adjusted to 50 g/l by adding toluene.

The toluenic solution of sotagliflozin (50 g/l) was maintained at a temperature of 70-75°C.

### Crystallization

The crystallisation of sotagliflozin was performed in a cascade of two continuous stirred-tank reactors (each of 600 ml).

The solution was taken to the reactor by pressure.

At the beginning, the first reactor was sown with 10% w/w of form II of sotagliflozin.

The first reactor was fed with the toluenic solution of sotagliflozin (50 g/l, 829 kg/m³) at 65-66°C with a flow of 1160 g/h (1400 ml/h), under stirring at 400 tr/min. The time of residence in the reactor was 30 minutes.

The medium obtained in the first crystallization chamber was harvested, was subjected to a wet milling, and was fed back in said first crystallization chamber.

The suspension of sotagliflozin obtained at the end of the first reactor was taken to the second reactor with a peristaltic pump.

The second reactor was fed with the suspension of sotagliflozin obtained at the end of the first reactor, at 25-26°C, under stirring at 600 tr/min. The time of residence in the reactor was 30 minutes.

The suspension obtained at the end of the second reactor was recovered for its isolation (filtration, washing, and drying).

### Filtration

A filtration of the suspension was performed at 25°C on a rotary pressure filter.

### Washing

A wash of the medium was performed at 25°C with 2 Volumes of toluene.

### Drying

A drying of the medium was then performed at 50°C under pressure below 50 mbar.

Form II of Sotagliflozin was crystallized with a yield of 97%.

An XRPD (X-ray diffraction) analysis confirmed that Form II of sotagliflozin was obtained.

### Example 3: Continuous process of preparation of crystalline form II of sotagliflozin with toluene in an intensified reactor

### Synthesis of sotagliflozin in toluene

Sotagliflozin was synthetized in an intensified reactor at 113°C under a pressure of 8 bar.

The reactor was fed with the solution of compound (A) in 10 Volumes of toluene with a flow of 1194.6 g/h and the methanolic solution of MeONa (0.6 equivalent of MeONa and 15 equivalents of MeOH) with a flow of 114.6 g/h, which were preheated at 113°C.

The time of residence in the reactor was 20 seconds.

The system was maintained at a pressure of 8 bar.

Sotagliflozin was synthetized with a yield of 99.7%.

Aqueous washing, dehydration, crystallization, filtration, washing, drying were performed according to example 2.

An XRPD (X-ray diffraction) analysis confirmed that Form II of sotagliflozin was obtained.

## Claims

1. A process for the preparation of the crystalline form II of sotagliflozin: 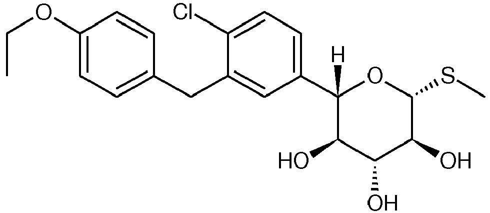 wherein said crystalline form II of sotagliflozin is directly obtained from the following compound of formula (A): 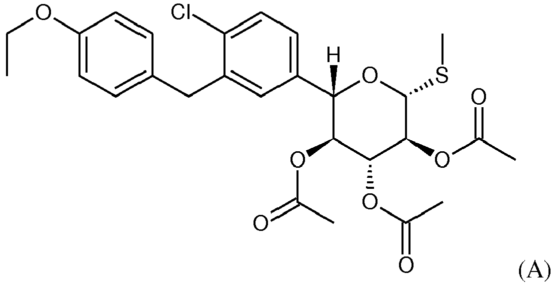 and by using toluene or xylene, or mixture thereof, as solvent medium for the crystallization.

2. The process according to claim 1, wherein said crystalline form II of sotagliflozin is directly obtained from the compound of formula (A) by using toluene as solvent medium for the crystallization.

3. The process according to claim 1 or 2, comprising the following steps:
a) reacting the compound of formula (A) with an alcohol, in a non-aqueous solvent medium comprising a base and including at least toluene or xylene or mixture thereof, and preferably at least toluene;
b) performing an aqueous washing of said solvent medium;
c) dehydrating said solvent medium;
d) crystallizing the form II of sotagliflozin; and
e) recovering the crystalline form II of sotagliflozin.

4. The process according to claim 3, wherein said solvent medium further comprises sodium methoxide and methanol.

5. The process according to any one of claims 3 or 4, wherein step a) is performed at a temperature below the boiling point of methanol.

6. The process according to any one of claims 3 to 5, wherein step a) is performed at a temperature below 65°C, and preferably below 63°C, at the atmospheric pressure.

7. The process according to any one of claims 3 to 6, wherein step c) is performed by evaporation.

8. The process according to any one of claims 3 to 7, wherein step d) is performed at the temperature of the crystallization of form II of sotagliflozin.

9. The process according to any one of claims 3 to 8, wherein step d) is performed at a temperature from 60°C to 70°C, preferably from 62°C to 67°C, and more preferably at 65 °C, at the atmospheric pressure.

10. The process according to any one of the preceding claims, wherein the formation of said crystalline form II of sotagliflozin is initiated with pre-existing crystalline form II of sotagliflozin.

11. The process according to any one of claims 3 to 10, wherein step d) is followed by filtration and washing, preferably with toluene or xylene, or mixture thereof.

12. The process according to the preceding claim, wherein the filtration and washing are followed with a drying step.

13. The process according to the preceding claim, wherein the drying step is performed at a temperature from 45°C to 65°C, and in particular from 50°C to 55°C.

14. The process according to any one of the preceding claims, wherein the crystalline form II of sotagliflozin is synthetized in a batch process.

15. The process according to any one of the claims 1 to 13, wherein the crystalline form II of sotagliflozin is synthetized in a continuous process.
